# EUROPEAN PATENT APPLICATION

(11) **EP 3 789 035 A1**
(43) Date of publication of application: **10.03.2021**
(21) Application number: 18924538.4
(22) Date of filing: 14.08.2018
(51) Int. Cl.: A61K 38/19, A61K 38/21, A61K 36/87, A61P 43/00, A61K 9/48, A61P 31/12, A61P 37/02, A61P 39/06, A61P 35/00

(54) **STABILIZED COMPOSITION**

(30) Priority: 27.06.2018 RU 2018123369
(71) Applicant: Chumburidze, Georgy Georgievich, Moscow 125008 (RU)
(72) Inventor: Chumburidze, Georgy Georgievich, Moscow 125008 (RU)
(74) Representative: Bucher, Ralf Christian
(86) International application number: PCT/RU2018/000531
(87) International publication number: WO 2020/005097

(57) **Abstract**

The claimed invention refers to the pharmaceutical field and represents a stabilized composition possessing antiviral, antitumor, immunomodulatory, actoprotective, antimutagenic and antioxidant activity. The composition can be used to support the methods of antitumor therapy, in particular, to eliminate postoperative complications, to recover after chemotherapy, radiation therapy and to prevent tumor disease recurrence.

The stabilized composition possessing antiviral, antitumor, immunomodulatory, actoprotective, antimutagenic and antioxidant activity is placed in an acid-resistant capsule containing calcium stearate, aerosil, microcrystalline carboxymethylcellulose, starch, lactose, grape seed powders, mannitol and low doses of lyophilized human recombinant gamma and tumor necrosis factor-thymosin alpha 1 comprising per 1 g of the composition(wt%):

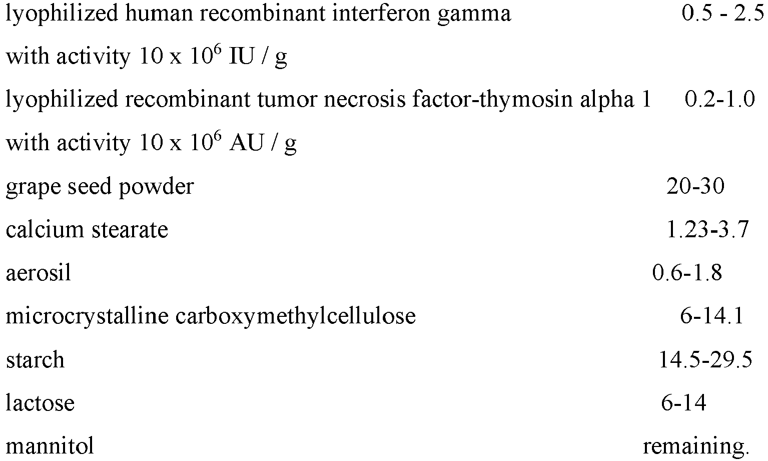

## Description

### Field of the invention

The claimed invention refers to the pharmaceutical field, represents a composition possessing antiviral, antitumor, immunomodulatory, actoprotective, antimutagenic and antioxidant activity and can be used to support the methods of antitumor therapy, including elimination of postoperative complications, recovery from chemotherapy, radiation therapy, prevention of recurrence of tumor disease.

### Background of the invention

A search for effective and safe drugs for the treatment of diseases characterized by high mortality or resulting in a severe disability of patients is one of the most important tasks of medical science. First of all, oncological diseases, sepsis and rheumatoid arthritis are among the diseases with unfavorable prognosis. In recent years, some steps forward in the treatment of patients with this pathology have been made due to the emergence of a deeper knowledge of the disease pathogenesis.

Currently, there is a large number of antitumor drugs in clinical practice, but the effectiveness of most of them is not high enough and therefore the issue of developing new more active drugs as well as the search for substances effective for the treatment of tumors with primary and acquired resistance to drug therapy, remains urgent. In this regard, the decision to promote a new substance with antitumor activity has been taken on the basis that the substance should meet the following criteria: a new mechanism of action, high antitumor activity, cytotoxicity selective with respect to certain in vitro tumor cell cultures and human tumor xenografts and the lack of cross-resistance to known drugs.

Continuous search for new drugs that could improve the quality of treatment of patients with oncological pathology has been carried out.

It is well known that oncological diseases are treated with chemotherapeutic preparations aimed at suppressing tumor growth and metastatic formations. In addition, the chemotherapeutic preparations themselves have pronounced negative properties with respect to healthy cells in the tissues of a human body. In particular, the genetic integrity of chromosomes is violated, which is manifested in a sharp increase in estimated chromosomal aberrations with the use of chemotherapeutic preparations. Another negative side effect is the disruption of metabolism of polysaccharides and lipids, as a result of which toxic products of metabolism accumulate both in cells and in the cytoplasmic liquid.

Thus, in order to reduce the negative effects of antitumor therapy, an additional complex health-improving effect on the human body is required.

Currently, much attention is focused on cytokines, biologically active factors of peptide nature which are produced by cells of the immune system and represents the main regulators of it. Initially, interferon was used exclusively as an antiviral agent and todayit is most successfully used in clinical therapy of viral or viral-related diseases. Interferon gamma also has significant prospects to be used in oncology. Thisis determined by its function to stimulate and control the activity of cytotoxic antitumor immunity. Mechanisms of antitumor protection of interferon gamma are realized by activation of macrophages, cytotoxic lymphocytes, induction of apoptosis of cancer cells, activation of antitumor immunity. Use of interferon gamma in oncology is associated with its immunomodulating activity. (O.I. Kiselev, F.I. Ershov, E.G. Deeva, "Interferon Gamma: A New Cytokine in Clinical Practice", Ingaron ", M-S-Pb, Dmitrade Graph Groups, 2007).

Thus, interferon gamma described in patent RU 2214832, published on 27.10.2003 is obtained from an Escherichia coli strain containing recombinant plasmid DNA pGIF315. The plasmid of a size of 3.15b.p. encoding recombinant human gamma- interferon with a size of 144 amino acid residues with a molecular weight of 16.9 kDa has the following properties: it contains promoter A2 between two restriction sites of endonuclease EcoRI and promotor A3 of T3 phage between restriction sites EcoRI and KpnI, a ribosome-binding site between restriction sites KpnI and XbaI, gene encoding synthesis of recombinant gamma interferon human between XbaI and BglII restriction sites and a transcription terminator between two restriction sites of XbaI, it also has an ampicillin resistance gene. Plasmid pGIF315 transforms E. coli strain having lon- and/or ompT mutations in protease genes.

A tumor necrosis factor - thymosin alfa1 (hereinafter TNF-T), represents a fusion protein consisting of TNF and thymosin alpha1.Construction of several fusion proteins is known from the prior art (patent for invention RU 2077586 published on 20.04.1997 and patent for invention RU 2055896 published on 03/10/1996).

A preparation produced under the trade name REFNOT ("Synthesis encoding recombinant plasmid DNA; method for construction and the preparation of recombinant fusion protein-α-tumor necrosis factor-thymosin-alpha 1") described in patent RU 2225443, published on10.03. 2004 is also known. A technology for production ofTNF-T substance implies microbiological synthesis of it by Escherichia coli SG 200-50 recombinant strain transformed by plasmid pThy. ProteinTNF-T is isolated and purified by column chromatography. TNF-T provides a directin vivo and in vitro antitumor effect on tumor cells of various lines. According to the spectrum of cytotoxic and cytostatic effects on tumor cells, the preparation corresponds to human tumor necrosis factor alpha (TNF), however, the total toxicity of it is 100-fold lower than that of TNF.

On fibrosarcoma cells L-929,a constructed fusion protein TNF-Thas a specific cytotoxicity 2.000.000 U per mg of protein and exhibits a strong immunostimulating effect. According to the spectrum and activity,cytotoxic and cytostatic effects of TNF-T on tumor cells are as high as those of TNF and in some cases they are even higher than those of human TNF. Moreover, TNF-T has a 100-fold lower total toxicity than TNF, which is confirmed by clinical trials. Phase I clinical trials conducted in N.N.Blokh in Russian Cancer Research Center, RAMS, Moscow, "Study of the safety, tolerability and efficacy of REFNOT in patients with squamous cell carcinoma of the head and neck", Department of Head and Neck Tumors (Prof. Lubayev V.L., MD, Head of the Department) showed low toxicity of REFNOT when administered intratumorally at doses up to 800 000 U (400 µg) per injection to oncological patients with braintumors.

Significant effectiveness for use in oncology can be achieved by combining interferon gamma and TNF. Synergism of TNF and a number of cytostatic preparations is known from preclinical studies (Balkwill, 1987, Takahashi, 1995, Mareel, 1988, Rosen 1992). However, due to the expressed systemic toxicity of TNF, these synergistic combinations have been little studied. In some studies, pronounced systemic reactions caused by vasoplegia were observed, which leads to poor vascularization and deterioration of bioavailability of the preparations, primarily TNF (Mayer, 1995, DeBlasi, 2005). It is not surprising that in 1986-1987 studies of recombinant TNF were discontinued because of severe systemic toxicity and the lack of satisfactory treatment effectiveness.

Interferon gamma and TNF-T are produced for intravenous, intramuscular or subcutaneous administration. As a rule, oral administration of IFN is not applied, since it is believed that under the action of proteolytic enzymes interferons are destroyed (inactivated) and are not absorbed in the native form. (Cantell and Pyhäla, J. Gen, Virol, 1973 20: 97-104; Gilson et al, J IFN Res, 1985 5: 403-408).

In recent years a number of reports on antiviral, antitumor efficacy of oral administration of low doses of recombinant IFN (mice and humans) in mice and positive immunological and clinical results in patients with AIDS, multiple sclerosis and type I diabetes with prolonged use of low doses of recombinant human interferon (10⁴-3 • 10⁴ IU) have appeared (Satoh Y., Kasama K., Yimin et al., Involvement of hypothalamic pituitary adrenal (HPA), infertility in 2-5 OAS by low dose oral administration of interferon, in. -J. Of Interferon & Cytokine Research 1999, v.19, p 125.Brod S.A,,PhanT., Katz S. et al., Ingest IFN α delays islet allograft rejecting. - J. of Interferon & Cytokine Research. 1999, v.19, p. 124 Brod S., Vriesendorp F.J., Ahn C. et al., Ingested IFNα, new MRI brain lesions in relapsing-remitting multiple sclerosis (RRMS), European Cytokine Network, 2000, v. 11, p. 154).

Development of side effects as a result of therapy is a limiting factor in the use of drugs. Most side effects are dose-dependent. Thus, it is not recommended to administer IFN-gamma in doses greater than 10⁸ IU due to the development of severe fever occurring 30-60 minutes after intravenous administration. Administration of large doses also leads to metabolic disorders (hyperpotassemia, increased levels of urea, creatinine and aspartic aminotransferase). Ischemic manifestations, arrhythmia are developed in a small number of patients.

Use of low doses of interferon for treatment of immunoresistant diseases, hyperallerginicity, autoimmune disorders, viral infections is described in WO1988 / 003411, published on May 19, 1988, US Patent 5,019,382, published on May 28, 1991, patent RU 2277932, published on 10.03.2006 and the patent for invention RU 2191594, published on October 27, 2002. Use of low doses of interferon in treatment of cancer in combination with radiation therapy or chemotherapy is described. IFN-gamma can be administered into the oral cavity, most preferably sublingually or buccally, or it can be administered parenterally. Interferon may also be administered per os, intranasally, for example, by inhalation of a powder or dispersed liquid droplets or locally.

Use of preparations at low doses for treatment of malignant is also known from the prior art (patent for invention RU 2475865, published on 10.11.2011). The effect of ultra-low doses was studied using a wide range of factors: antitumor agents, hormones, immunomodulators, antioxidants, etc. (Burlakova.E.B., Vesti, RAS 1994 vol. 64. Nº5, p.425).

It is generally accepted that treatment of human diseases is carried out by administering a dose of compounds chosen for the inhibitory effect of the causes and / or symptoms of the correspondingstate. However, this principle is sometimes ineffective and often there are side effects, such as intolerance or allergic reactions. Therefore, at present, attempts are being made to create new drugs that have a curative effect and a minimal side effect.

Treatment of immune-resistant disease with a low dose of interferon is well known. Thus, tumor diseases, hyperallerginicity, autoimmune disorders, viral infections are treated by administering interferon at a dose of about 0.1-5 IU/pound per day by contacting interferon with the pharyngeal mucosa. Interferon is administered in solution or in a new solid dosage form adapted to be dissolved in saliva when it enters the mouth (US5019382, A, May 28, 1991).

Use of low doses of interferon is known for treatment of neoplastic diseases (US Pat. No. 5,019,382, published on May 28, 199). Tumor diseases, autoimmune disorders, viral infections are treated by administration of interferon at a dose of 0.2 to 12 IU/kg per day. Interferon is administered in a liquid or a solid dosage form adapted to be dissolved in saliva when it enters the mouth.

The known resorption tablet described in RU 2277932, published on 10.03.2006, has an antiviral effect comprising an amount of 1/5-1/20 of its single therapeutic dose and one of recombinant human interferons in amount of 500-1000 IU in a ratio of 2: 1-3: 1 and auxiliary substances, while the tablet has a mass of 0.6-0.7 g, diameter of not less than 12 mm and a height of 2-3 mm.

A preparation that exhibits an immunomodulatory, antimicrobial, antioxidant and regenerative effect is described in patent RU 2255760, published on July 10, 2005. This invention proposes the preparation and its various dosage forms for oral, parenteral and local administration (tablets, sprays, solutions, gels) for administration to mucous membranes (suppositories) based on natural and recombinant interferons (alpha, gamma , beta), lysozyme and emoxipin. Emoxipin in this product serves as an antioxidant. However, these properties of emoxipin are mostly exhibited in the conditions of hypoxia. The preparationin a tablet formcontains a large amount of lactose (0.3 g). Lactose is known to cause digestive disorders due to reduced activity or the lack of the corresponding enzyme lactase in many patients. When producing the preparation in the form of tablet interferon may be destabilized, since a significant increase in temperature occurs during tableting under pressure.

A pharmaceutical composition in the form of capsule possessing antiviral and antimicrobial activity disclosed in patent RU 2292907 published on February 10, 2007 is the most advantageous technical solution of the problem. The composition represents a mixture for forming a capsule content, contains recombinant human interferon-a, a swelling agent and target additives and the following ratio of components in 1.0 g of the composition:
humanrecombinant interferon-a 10000-3000000 ME
polyethylene glycol 4000-6000 0.0001-0.5
polyglukin 0.001-0.1
structure-forming agent 0.01-0.8
swelling and dispersing agent 0.001-0.1
target additive remaining.

Preparations of recombinant and natural cytokines are traditionally used at high doses parenterally (intramuscularly, subcurtaneously, intravenously) for treatment of hepatitis B and C, multiple sclerosis and various oncological diseases. And it is known that parenteral administration of drugs at high doses causes, as a rule, the development of influenza-like syndromes, such asa fever, arthralgia, catarrhal phenomena anda headache. Per os administration of IFN drugs is not applied, since it is believed that proteolytic enzymes of LCG interferons are destroyed (inactivated) and are not absorbed in the native form.

The present invention is aimed at designing a stabilized composition possessing antiviral, antitumor, immunomodulatory, actoprotective, antimutagenic and antioxidant activityproviding a good tolerability, minimal side effect and having activity for use in complex treatment of cancer diseases as well as improving the quality of life of the patient. The stabilized composition should retain its properties when stored under normal conditions and being frozen, and also should withstandshort-time exposure to high temperature.

### Summary of the invention

The claimed invention is aimed at achieving the following technical results:
- achievement of the stabilizing effect of cytokines, due to the optimally selected composition of components and their quantity, which allows the storage of interferon gamma and TNF-T under normal conditions without loss of activity
- preparation of a stabilized composition based on human recombinant interferon gamma and TNF-T possessing high antitumor, antiviral, antioxidant, immunomodulating, actoprotective, antimutagenic activity
- expansion of the storage temperature range of interferon gamma and TNF-T without loss of activity
- minimal side effects providing by the composition components, including subfebrile conditions and dyspeptic disorders, which makes it possible to use the product in complex therapy in the treatment of oncological diseases, including anti-relapse treatment, for the prevention of cancer and for the improvement of the quality of life of people and animals.

These technical results can be achieved by using a stabilized composition that possesses antiviral, antitumor, immunomodulatory, actoprotective, antimutagenic and antioxidant activity. In addition to direct therapeutic effect the stabilized composition provides good tolerability, minimum side effects and also improves the quality of life of humans. And the composition does not demand specific storage conditions (it can be stored at room temperature).

In this case, the composition has a shelf life acceptable for use in pharmaceutical manufacture and it is also technologically feasible for production.

The claimed stabilized composition possesses antiviral, antitumor, immunomodulatory, actoprotective, antimutagenic and antioxidant activity. It represents an acid-resistant capsule containing calcium stearate, aerosil, microcrystalline carboxymethylcellulose, starch, lactose, grape seed powder, mannitol and lyophilized human recombinant interferon gamma and tumor necrosis factor-thymosin alpha 1in low dosescomprising per 1 g of the composition:(wt%):

| | |
|---|---|
| lyophilized human recombinant interferon gamma with activity 10 x 10⁶IU/g | 0.5 - 2.5 |
| lyophilized recombinant tumor necrosis factor-thymosin alpha 1 with activity 10 x 10⁶U/g | 0.2-1.0 |
| grape seed powder | 20-30 |
| calcium stearate | 1.23-3.7 |
| aerosil | 0.6-1.8 |
| microcrystalline carboxymethylcellulose | 6-14.1 |
| starch | 14.5-29.5 |
| lactose | 6-14 |
| mannitol | remaining |

Lyophilized human recombinant interferon gamma is constructed from the substance of interferon gamma isolated from E. coli strain transformed with plasmid pGIF315.

Lyophilized recombinant tumor necrosis factor-thymosin alfa is constructed from the substance of tumor necrosis factor-thymosin alpha 1 isolated from Escherichia coli producer strain transformed with plasmid pThy316.

### Brief description of the drawings

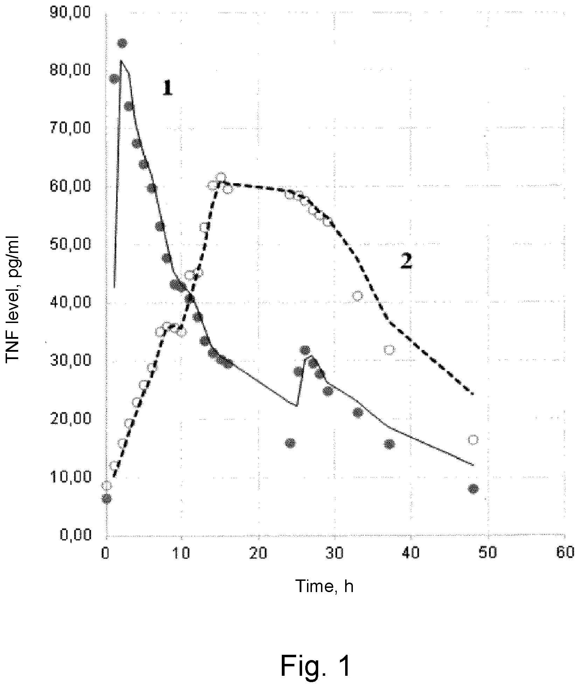

The essence of the claimed group of inventions is explained by the drawing given below. A diagram comparing the dynamics of concentrations of TNF in venous blood, where 1 is the change in the concentration of TNF after application of Refnot+ Ingarone, and 2 is the change in the concentration of TNF after the application of thermostable composition, is presented in FIG. 1.

### Detailed description of the preferred embodiments

In order to obtain the claimed product, interferon gamma "Ingaron" manufactured by OSC "NPP" Farmaklon "and Sigma and recombinant tumor necrosis factor-thymosin alfa1" REFNOT "manufactured by OSCRefnot-Pharm were used.

Dosage forms of TNF-T in combination with interferon gamma that can be used for treatment of diseases, including oncology have minimal side effects and are not known in the art.

Protein-based drugs should often have a high concentration to achieve therapeutic effect.This creates many problems, including the production, preservation of the stability, activity, which is especially characteristic of proteins.

Therefore, the limits of the quantitative content of proteins as well as the quantitative and component composition necessary for their stabilization in these boundaries were determined experimentally.

It has been unexpectedly discovered thatin the claimed amount of the invention it is the grape seed powder, lactose, mannitol, calcium stearate, aerosil, microcrystalline carboxymethylcellulose that can reduce or prevent both the effect of external factors and negative interaction / reaction between the biologically active proteins contained in the drug by forming a stable complex.

The present invention is mainly aimed at designing low-dose cytokine compositions and their use for prevention and treatment of various diseases, including cancer treatment in combination with radiation therapy or chemotherapy.

The present invention allows for the use of low doses of TNF-T (from 4000 to 20000 AUper capsule) and interferon gamma (from 10.000 to 50000 IU per capsule). The stabilized composition of the present invention in the form of capsules can be used in combination with other therapies, for example in the case of cancer treatment it can be used in combination with surgical removal of tumor, therapy or radiation therapy or chemotherapy, or in the case of microbial diseases it can be used in combination with antibiotics. The produced capsule is acid-resistant or ithas an acid-resistant coating. The point is that the injection form of administration is accompanied by local negative phenomena, such as painful irritation and inflammation at the injection site, induration of subcutaneous layers leading to a decrease in drug absorption. All this requires a constant change of the injection site and provides the risk of infection. The proposed oral form has no such draw backs, which allows the patient to take the drug independently and improves the quality of life of the patient. Use of acid-resistant capsules allows the product to avoid proteolytic cleavage in the stomach. In addition, the capsule allows the powders to be simultaneously compact but not compressed, thereby combining the advantages of tablets (compactness) and powders (rapid dissolution and absorption of the drug), that is, the capsule provides increased bioavailability and stability of the components.

In the known clinical treatment regimens, separate injection of TNF-T and gamma-interferon is used. In this case, the drugs are used successively and their intake is separated in time.

In the medicinal form that we offer, both biologically active compounds enter the body simultaneously, which enhances the synergistic effect. As a result, use of the claimed form allows for reducing the dosage of the main active substances while maintaining the overall cytokine effect (an increase in the expression of differentiation clusters on the surface of T and B lymphocytes and an increase in the expression of histocompatibility genes of the first class were shown) as well as for reducing the inflammatory intoxication.

Due to the simultaneous gradual (oral administration) low-dose intake (maximum 50.000 IU per one capsule and 20.000 AUper one capsule), interferon gamma in combination with TNF-T has a mild cytotoxic effect and does not exhibit toxicity registered when using monocomponent drugs administrated by injection. TNF-T and interferon gamma contribute to the establishment and maintenance of a sufficiently high (therapeutic, but not toxic) level of TNF in human blood, which provides a self-correction of the immune processes in the body and activates the destruction of tumor tissue in the absence of undesirable reactions.

Using a simple mixture of lyophilized TNF-T powders and interferon gamma when administered to animals per os(capsules No. 5), it was noted that the amount of the pharmaceutically active component entering the blood and tissue of animals was not more than 5% of the total amount of the administered product. The studies were carried out by ELISA. After using enteric-soluble capsules with a stabilized composition, it was found that the concentration of active components in the same biological objects was on average 0% of the administrated quantity in the sample and up to 35% in some cases. Thus, it has been shown that the introduction of a grape seed cake in the mixture increases the bioavailability of the main components.

Owing to the optimally selected components of the claimed composition, it was possible to increase the bioavailability of the active components and due to the combination of all the claimed components it was possible to obtain a technologically stable product.

In order to prepare the stabilized composition, samples of lyophilized cytokines were prepared. The samples were mixed with grape seed powder, aerosil, starch, calcium stearate, microcrystalline carboxymethylcellulose, lactose and mannitol. Then, the obtained composition was placed into acid-resistant capsules No. 1, 0.2 g each.

Protein amounts in lyophilized human recombinant interferon gamma and TNF-T were determined by solid-phase enzyme-linked immunosorbent assay using commercial kits of reagents manufactured by Vector Best Company (Novosibirsk). In the kits, a "sandwich" version of the solid-phase enzyme-linked immunosorbent assay was used. In order to implement this variant, two monoclonal antibodies with different epitope specificities were used for each component. When preparing samples for analysis, the content of the capsule was placed in a test tube and 2 ml of sodium phosphate buffer was added. The content then was mixed and allowed to stand for 30 minutes. The resulting suspension was sterilized. The samples were used for testing.

The biological activity of interferon gamma and TNF-T in the capsule and the stability of the capsule were determined using Vero cell line for interferon gamma and L929 line for TNF-T.

In order to evaluate storage stability, methods for monitoring the biological activity of the claimed composition at various storage temperatures were used. The methods implied testing of antiviral activity of interferon gamma and cytotoxic activity of TNF-T. For this, 2 series of the stabilized composition prepared according to Examples 1, 2, 3 (see below)were used. Each of the compositions was divided into three equal groups and stored under different temperature conditions: under standard conditions (+ 25 °C), at plus 30 °C and at plus 35 ° C.

During the first month of testing, control samples from different groups were collected weekly. Within six months the sampling was made every month. Subsequently, analytical samples were taken every three months.

Evaluation of the antiviral activity of interferon gamma during storage was performed using Vero cell culture an in vitro test system. The virus of vesicular stomatitis (VVS) was used a test virus. VVS was taken at a concentration of 10 TCD₅₀(tissue cytopathic dose). Sample preparation was carried out by dissolving 200 mg of the composition of the first and second series, respectively, in 1 ml of water for injection.

In order to investigate the antitumor activity of tumor necrosis factor-thymosin alpha 1, L-929 cell line was used.

In the test system, the concentration of cells in both cases was (2-2.5) x10⁶. In the case of evaluation of antitumor activity, a visual method of counting monolayer damages as a result of the harmful effect of tumor necrosis factor thymosin alpha 1 on the cells of this monolayer was used.

As reference samples, the following specially prepared lyophilized mixtures were used: gamma interferon recombinant human substance in phosphate saline buffer with the activity of 100.000 IU / ml; the substance of tumor necrosis factor thymosin alpha 1 in phosphate saline buffer with the activity of 50000 U / ml. It was difficult to evaluate the activity of a simple mixture of interferon gamma and TNF-T in a phosphate-buffered saline solution by the above methods. This is probably due to the complex effect of the components on the cell lines leading to a strong distortion of the results. Therefore, in order to quantify the activity of the components, a method of inhibiting the component, whose activity control was not performed, was applied. Thus, when controlling the activity of interferon gamma on Vero cell line, TNF-T was pre-inhibited by specific monoclonal antibodies. Similarly, when testing the activity of TNF-T on L969 cell line, the activity of interferon gamma was inhibited.

Specific activity of cytokines was determined according to the procedure described below.

Interferon gamma activity was determined using Vero cell culture in the presence of monoclonal antibodiesspecific to TNF or TNF-T.

For the test, the contents of 10 capsules were combined. From the resulting volume of the powder, an aqueous extraction was made and filtered through a 0.22-µm acetate cellulose filter.

First, successive ten-fold dilutions of 1:10, 1: 100, 1: 1000were prepared, after which two-fold dilutions of the investigated samples of the stabilized composition and WHO interferon gamma international standard(whose activity is expressed in international units, IU) in growth medium with the addition of monoclonal antibodies to TNF (or TNF-T) were made as follows: 1: 2000, 1: 4000, 1: 8000, 1: 16000, 1: 32000, 1: 64000, 1: 128000. The medium was removed from the plate with the monolayer of cells and successive dilutions of the samples of the stabilized composition were added, 0.1 ml to each well. At least four wells with a cell culture were used for each dilution of the stabilized composition sample. Four wells with the culture were used as control. In addition, 16 wells were used to control the dose of the indicator virus. 0.1 ml of growth medium was added to these wells. Inoculated and control cell cultures were incubated for 1 day at (37 ± 1.0) ° C in the atmosphere of (5.0 ± 0.5)% CO² under virus dose control.

Results of interferon activity determination were recorded when the dose of introduced virus corresponded to 100 TCD₅₀. If the virus dose corresponding to 100 TCD₅₀was calculated on the basis of the results recorded 24 hours after infection, interferon titration could also be performed after 24 hours under the microscope. The record of the test results was possible if there were no signs of degeneration in the control culture. The interferon titer was taken as the inverse of the dilution of the stabilized compositionsample, in which the cell culture in 50% of the wells was completely protected from the cytopathic effect of the virus. Recalculation of activity in IUwas carried out according to the known formulas.

TNF-T activity was determined on L929 cell culture in the presence of specific monoclonal antibodies to interferon gamma.

For the test, the contents of 10 capsules were combined. From the resulting volume of the powder, an aqueous extraction was made and filtered through a 0.22-µm acetate cellulose filter.

First, successive ten-fold dilutions 1:10, 1: 100, 1: 1000were made, after which two-fold dilutionsof the tested samples of the stabilized composition in a growth medium with the addition of monoclonal antibodies to human IFN-gamma were performed as follows: 1: 2000, 1: 4000, 1: 8000, 1: 16000, 1: 32000, 1: 64000, 1: 128000. The medium was removed from the plate with a monolayer of cells and 0.1 ml of successive dilutions of the stabilized composition samples and 0.1 ml of the growth medium with actinomycin A were added to the wells. At least four wells with a cell culture were used for each dilution of the sample of the stabilized composition. Four wells with the culture wereused as control. The cell culture was incubated for 1-2 days at (37 ± 1.0) ° C in the atmosphere of (5.0 ± 0.5)% CO₂.

The results were recorded after 24 hours (in advance) if there was no microscopic evidence of monolayer degeneration in the control wells. The final recording of the results was carried out after 48 hours.

The monolayer was washed from the dead cells with a sterile saline solution and stained with a 0.2% solution of crystalline violet. The results recordwas performed visually under the microscope or spectrophotometrically using a plate spectrophotometer at a wavelength of 540 nm.

The amount of sample of the stabilized composition causing 50% cell damage (or a two-fold drop in the optical density with respect to the optical density of the control wells) under standard conditionswas taken as 1 IU.

Calculation of the activitywas carried out according to the known formulas.

As a result of the claimed composition stability evaluation during storage it has been found that:
1. The initial activity of the prepared reference solutions corresponds to the expected and in value is equal to the reference samples containing any of the active substances under standard conditions.
2. When stored under specified conditions during the first month, control samples containing only human recombinant interferon gamma, regardless of the buffer composition, lost up to 70% of the activity when stored at room temperature and were completely deactivated at temperatures of 30 ° C and 35 °C.
3. When stored under specified conditions during the first month, control samples containing only tumor necrosis factor thymosin alpha 1 lost up to 20% of their activity when stored at room temperature, up to 40% when stored at 30°C and were deactivated to less than 50 % of the initial activityunder prolonged exposure to high temperatures. Thus, TNF-T is more stable than interferon gamma, which is probably related to its structure.
4. The stabilized composition retained the initial activity of its protein components during the first month at the initial level under all storage temperatures.
5. During the storage for the first three years, the stabilized compositions retained their thermostability at plus 25°C and at plus 30°C. When stored for more than three years at 35°C, the activity loss up to 10% of was recorded.

Thus, it has been confirmed that the claimed composition is stable and retains its properties when stored at ambient temperature up to plus 30°C for 3 years.

In order to confirm the claimed properties of the stabilized composition, namely, antiviral and antitumor activity, experiments were performed on in vivo models (rats, mice). As a result, antimutagenic, immunomodulating, antioxidant and actoprotective properties of the claimed stabilized composition were additionally revealed. The antiviral activity of the stabilized composition was demonstrated on cell models in vitro.

In order to determine the antitumor activity of the invented stabilized composition, BDFI mice male weighing 19-22 grams with transplacedepidermal Lewis lung carcinoma (LLC) of a size of about 1.6 cm³ were used. In the experiment, three groups of animals, 20 individuals each, were used. The control group (G1) was given a placebo placed in capsules No. 5. The experimental group (G2) was given the claimed stabilized composition per os. Experimental group G3 received therapy according to scheme Refnot + Ingaron intramuscularly. The dosage of the test composition was preliminarily corrected taking into account the volume of capsules and the number of units of activity per animal body weight. The growth of LLCs in all groups was estimated after a single and multiple (daily) capsule administration per os using a probe.

In order to assess the antioxidant activity of the claimed stabilized composition, the dynamics of accumulation of malonicdialdehyde (MDA) was studied. Previously, G3 animals were typed according to the level of background activity of natural antioxidants, such as glutathione, ascorbate, tocopherol.

In order to assess the tumor state, the level of alpha fetoprotein (AFP) was used.

When comparing the indicated indices in animals receiving therapy of TNF-T and gamma (Refnot + Ingarone) intramuscularly and capsules No. 5 combined with the claimed stabilized composition given per os, asustained and prolonged decrease in levels of MDA and AFP was demonstrated in G2 animals. At the same time, in G1 animals, the AFP level decreased more slowly and the MDA concentration remained at the initial level during the entire period of therapy.

Based on the above study, it can be concluded that the claimed stabilized composition administered per os exhibits antioxidant and antitumor activity, most likely due to the synergistic effect of interferon gamma, tumor necrosis factor thymosin alpha 1 and grape seed powder.

When processing the data obtained, it was found that the use of components in the lyophilized form with low cytokine content leads rather to an increase than to a decrease in their specific activity and effect on mice. However, the studies have shown that such activity of cytokines is manifested only in the claimed stabilized composition that in addition to the molecules of interferon gamma and tumor necrosis factor-thymosin alfa1 also contains a grape seed cake. In a series of additional studies, when the effect of only a mixture of active protein components was evaluated without the presence of grape seeds, the effect of increased activity was not observed. With a decrease in the dose of active substances, the total effect in this case also decreased to practically the levels demonstrated by control groups of animals.

As the studies have shown, interferon gamma and TNF-T proteins possess different physical-chemical properties and the design of combined dosage forms with these proteins involves difficulties. Perhaps, this is due to the fact that the optimal conditions for the manifestation of biological activity of interferon gamma and TNF-T are not identical. For example, human recombinant interferon gamma is stable and is stored at pH 6.0-6.5, whereas TNF-T works best in neutral and slightly alkaline conditions (pH 7.0-7.5). The fact that different proteins behave differently is related to their amino acid composition (primary structure) and physical-chemical properties.

However, when designing the claimed invention, a number of experiments were carried out, which allowed for overcoming the existing difficulties. During the experiments, it was unexpectedly found that the introduction of the grape seed cake together with lyophilized interferon gamma and TNF-T significantly enhances the penetration of cytokines through the intestinal wall, thereby increasing the bioavailability of the active components. The experiments have shown that in the claimed composition, grape seeds, recombinant interferon gamma and TNF-T have a pronounced synergistic effect, with the specific activity of each cytokine being equal to the theoretical value.

It is known that introduction of cytokines (interferon gamma and TNF-T) causes an immune response that is accompanied by activation of free radical oxidation, which, in turn, deactivates the activity of cytokines. However, in the claimed composition containing cytokines in combination with grape seeds, no deactivating effect is observed, which makes it possible to suggest the existence of an antioxidant effect. Apparently, this is due to the fact that in this composition the biologically active substances of the grape seeds act as "traps of free radicals", because these free radicals adversely affect interferon gamma and TNF-T. Therefore, it was possible not only to remove side effects, but also to obtain a long-term synergistic effect. This assumption was confirmed by the experimental data given below.

In order to confirm the antitumor property of the claimed composition (in the form of a capsule), an experiment was conducted in which male mice of BDFI line weighing 19-22 g with transplacedepidermal Lewis lung carcinoma (LLC) of a size of about 1.6 cm³ were used. The animals were subdivided into three groups, 20 individuals each.

Additionally, experiments were conducted to identify the possibility of using the claimed stabilized composition for the prevention of oncological states (antimutagenic properties).

During the study of antimutagenic properties of the claimed stabilized composition, a well-known and widely used method for estimation of chromosome aberrations was applied to control the effect of the claimed composition. Cyclophosphamide that is appliednot only as a chemotherapeutic agent in the treatment of cancer, but also as a reference mutagen was usedas a positive control.

In this experiment, cyclophosphamide was administered to Balb-c mice. Capsules No. 5 prepared according to Example 1 were used. Preliminary they were moistened with water to make theircoat soft and easy to pass through the esophagus of the animals. After the control time, the animals were euthanized and biological material was collected for laboratory testing by the method of chromosome aberration estimation. All animals were subdivided into three equal groups. Group 1 animals were not subjected to any influences and were used as a control. Group 2 mice were treated only with cyclophosphamide and the animals of Group 3 were additionally given the stabilized composition prepared according to the claimed invention.

As a result, it was found that a sharp increase in the estimatedlevel of chromosomal aberrations was observed in the animals from the positive control group, whereas in the group receiving both the mutagen and the claimed stabilized composition this level was significantly lower, although it exceeded the control values.

Thus, it has been experimentally confirmed that use of the claimed stabilized composition reduces the level of chromosomal aberrations resulting from the effect of the chemotherapeutic drug cyclophosphamide. Thus, the claimed stabilized composition demonstrates antimutagenic and antioxidant properties.

The experimental conditions (acute phase) did not allow for estimating the overall effect of the claimed stabilized composition on the organism of experimental animals. Therefore, an attempt was made to evaluate this combined effect experimentally.

Oncoprotective properties of the invented stabilized composition were also revealed, as confirmed by the studies aimed at determining the expression of leukocyte integrins LFA-1 and Mac-1 on immunocytes and at estimating serum levels of IL-6 and IL-10 using mice of inbred CBA line genetically predisposed to the development of spontaneous hepatocarcinomas and at possible correcting these indicesusing the claimed composition. CBA inbred male mice (subline CBA / Lac Y) were used. This line is a classic model of genetic predisposition to liver tumors with a high risk of their occurrence. The first spontaneous hepatomas appearin CBA male mice at the age of 6 months and occur 7 times more often than in females. In late ontogenesis in males at the age of 18-22 months hepatocarcinomas are diagnosed in 100% of cases. It is known that the decrease of leukocytic integrins LFA-1 and Mac-1expression on immunity effectors in parallel with an increase in the level of IL-6 and IL-10 in blood serum is observed in CBA male mice.

Use of the claimed compositionin preventive regimenin early ontogenesis covering briefly the "critical" period of liver tissue development (7-10 days) contributes to the long-term increase in the number of CD11a + and CD11b + lymphocytes expressing LFA-1 and Mac-1 molecules, respectively, as compared to control animals.

Male CBA / Lac-Y mice at the age of 4-5 months weighing 18-22 grams were used. All animals were subdivided into two groups, one of which was used as a control. Animals of the control group weredaily given a placebo placed in capsules No. 5. Animals of experimental group were given the claimed stabilized composition also placed in capsules No. 5. An oral probe was used to administer the capsules. Capsules with the claimed stabilized composition were given according to the following scheme: the substances were given during one month with two months interval. The total duration of the experiment was 12 months, during which at least five animals from each groupwere taken at equal intervals to assess the blood indices recorded in the experiment.

As a result, it was found that use of the stabilized composition in the preventive regimen, including early ontogenesis, contributes to a long-term increase in the number of CD11a + and CD11b + lymphocytesexpressing the molecules of LFA-1 and Mac-1, respectively, as compared to control animals. Thus, it has been established that use of the claimed formulation for preventive purposes does indeed reduce the level of hepatocarcinoma formation, i.e. the claimed composition has an oncoprotective effect.

During the study of general therapeutic effects of the stabilized composition prepared according to the examples given below, a method of artificial stress was used in two experimental groups of animals. Animals of Group 1 were given the composition with feeding, where as animals of Group 2 were not given the composition. In order to assess the impact of the measures taken, the animals of the same line of Group 3 were not subjected to stress and did not receive the claimed stabilized composition. As stress factors, mechanical intervention was used without causing physical injury to animals. The lighting regime was changed drastically, cages with experimental animals were subjected to mechanical actions, the animals were transferred from one cage to another and so on.

As a result, it was found that in animals of the group that did not receive the stabilized composition, but underwent stress,the level of the detected chromosomal aberrations was increased as compared to the control animals. The more intense the stress was, the more deviations in the recorded index were observed. However, this indexwas stabilized and even slightly declined with time. Apparently, the latter is associated with the natural mechanisms of adaptation of the animal organism to the same type of stress effect.

In the group, where mice were given the stabilized composition and were subjected to stress, it was observed that the animals wereadaptedto stress much more quickly and quickly calmed down. Although the recorded level of chromosomal aberrations in the animals of this group was significantly lower than in the mice of the previous group, but it was significantly lower than in the animals of control group.

Thus, it was found that the stabilized composition in addition to reducing the level of spontaneous chromosomal aberrations in the experimental line of mice, favorably affects the overall level of their vital activity, which in turn indicates an improvement in the quality of life of the animals.

The claimed stabilized composition can be used as a prophylactic agent, since it possesses antimutagenic and antioxidant activity.

Based on the performed studies and on theoretical speculations, there isa reason to believe that the claimed stabilized composition may have a wide spectrum of antitumor and antiviral activity.

This assumption was verified in clinical trials.

A number of randomized clinical trials involving immunodeficient diseases, such as disseminated skin melanoma, breast cancer, cervix, large intestine, head and neck and other localizations, and HIV / AIDS in combination with pulmonary tuberculosis have been conducted for this purpose.

Thus, a controlled randomized clinical trialon the effect of the stabilized composition (capsules for administration per os) on human immune system in comparison with the injectable drugs Refnot (tumor necrosis factor-thymosin alfa1 recombinant) and Ingaron (human gamma recombinant interferon) has been performed. The trial involved 390 patients with oncological diseases of various localizations and stages. The trial showed that after 2 months efficiency of the therapy did not statistically differ in patient given the claimed composition. According to RECIST criteria, positive dynamics, that is complete or partial remission and stabilization of the process, was observed on average in 56% vs. 53% of the patients and in control groupcomplete or partial remission was noticed in 42% vs. 36%). However, after 6 months a significantly greater number of patients were in the phase of clinical remission (72% vs. 48%).

The performed trials have shown that the stabilized composition contributes to:
1) decrease in the frequency of development of toxic manifestations in response to treatment;
2) increase of the effectiveness of antitumor immunomodulatory therapy manifested in the prolongation of the average duration of remission.

A controlled randomized clinical trial of the effect of the stabilized composition (capsules for per os administration) on human immune system in comparison with injectable drugs Refnot (tumor necrosis factor-thymosin alfa-1 recombinant) and Ingaron (human gamma recombinant interferon) has been performed according to the following scheme.

32 patients with breast cancer, 38 patients with ovarian cancer, 36 patients with cervical cancer, 44 patients with lung cancer, 36 patients with hepatocellular carcinoma, 34 patients with bile duct cancer, 58 patients with colorectal cancer, 32 patients with esophageal cancer, 38 patients with stomach cancer, 36 patients with pancreas cancer were involved in the trial.

Treatment regimen was as follows: patients of Group 1 were given the stabilized composition per os, 2 capsules 2 times a day without intervals; patients of Group 2 were given Refnot 200.000 IU subcutaneously once a day and Ingaron 500.000 IU subcutaneously once a day every other day for 10 days (5 injections of each drug) before and during the intervals between chemotherapy cycles. Both groups were comparable in terms of demographic parameters, distribution of patients by localization and stages of the oncological process as well as in terms of other significant factors (initial level of TNF, concomitant pathologies, regimens of antitumor therapy, etc.).
Subgroup 1. Patients with unresectable locally advanced or metastatic breast cancer of Stage III-IV at the age of 37-71 years (N = 32) were treated with a combined method. First, drug therapy + immunotherapy (the stabilized composition vs. Refnot + Ingaron) was performed, after which clinical efficacy of the therapy was evaluated. In case of inefficiency, alternative drug therapy + immunotherapy were tried then the effect was re-evaluated. In the case of reducing the tumor size to are sectable state a surgery was performed. After the first-linetherapy 3 patients in each group (19%) were operated. After the second -line therapy 4 more patients in the subgroup studied and 5 patients in the control group were operated. The final effectiveness of preoperative therapy in both groups did not differ significantly (44% and 50%, respectively). After resection, immunotherapy was performed, followed by postoperative radiation therapy and drug therapy according to indications. The evaluation of long-term results of treatment was conducted 6 months after the beginning of treatment and showed that patients who received the stabilized composition remained in the phase of clinical remission after the expiry of half a year (38% vs. 19%) twice as often
Subgroup2. Patients with common ovarian, uterine tube or primary peritonial cancer of stage IIb-IV at the age of 46-68 years (N = 38) who had not previously received anti-tumor therapy underwent immunotherapy (the stabilized composition vs. Refnot + Ingarone), then surgical intervention and further neoadjuvant polychemotherapy with the addition of immunotherapy during the interval between cycles. The overall positive effect of the therapy (stabilization, complete or partial regression of the tumor) after 6 months of observation was 97% in the patients of the tested group, whereas in the patients of the control group this index was 69%.
Subgroup3. Patients with squamous cell carcinoma of the cervix Ia1-Ib1 at the age of 36-67 years (N = 36) underwent preoperative immunotherapy (the stabilized composition vs. Refnot + Ingarone), followed by hysterectomy and postoperative immunotherapy with adjuvant chemotherapy with cisplatin according to indications. Based on the results of the observation within 2 months from the beginning of therapy, the majority of patients with insignificant intergroup differences (92% and 83%) showed high efficiency of complex therapy, following the observation for 6 months all patients receiving the stabilized composition remained in the phase of clinical remission (100% vs. 81%).
Subgroup4. The trialinvolved patients with non-small cell lung cancer of Ib-IIIa stage at the age of 34-73 years (N = 44), who subsequently underwent combined therapy: resection + adjuvant chemotherapy based on cisplatin supplemented with immunotherapy (the stabilized composition vs. Refnot + Ingaron) before the operation and between the cycles of chemotherapy. After 6 months, 20 patients of 22 (91%) given the stabilized composition were in the state of clinical remission (vs. 81% in the control group).
Subgroup5. Patients with unresectable common hepatocellular carcinoma of stage B or C at the age of 52-71 years (N = 36) were given palliative medication with sorafenib (800 mg / day) supplemented with immunotherapy (the stabilized composition vs. Refnot + Ingaron) before treatment and between the cycles of chemotherapy. After 6 months of observation, the absence of disease progression was noticed in the majority of patients receiving the stabilized composition (91% vs. 58%).
Subgroup6. Patients with unresectable bile duct adenocarcinoma at the age of 67-83 years (N = 34) were given combined palliative chemotherapy (gemcitabine + 5-fluorouracil) with the addition of immunotherapy (the stabilized composition vs. Refnot + Ingaron) before treatment and between chemotherapy cycles. Both groups showed significantly ineffective treatment after 2 months of maintenance treatment (39% vs. 38%), in 6 months of observation the use of the stabilized composition was more effective (78% vs. 38%).
Subgroup7. Patients with metastatic colorectal cancer (adenocarcinoma) at the age of 41-71 years (N = 58) who had not previously received anti-tumor therapy underwent immunotherapy (the stabilized composition vs. Refnot + Ingarone), then chemotherapy according to the FOLFOX6 regimen with an interval of 2 weeks, during which the immunotherapy was continued. The combined treatment was effective in more than half of the patients (55%) in both groupsafter 2 months. After 6 months, the effectiveness of therapy in the trial group reached 68% (vs. 57% in the control subgroup).
Subgroup8. Patients with unresectable squamous esophagus cancer without severe dysphagia at the age of 49-74 years (N = 32) who had not previously received anti-tumor treatment underwent immunotherapy (the stabilized composition vs. Refnot + Ingarone), then palliative chemotherapy (cisplatin + capecitabine for 2 weeks) with 3-week intervals, during which immunotherapy was continued. After 2 cycles of chemotherapy, the objective response rate was 36% and 38%, after 6 cycles it was 87% and 36% (the stabilized composition vs. Refnot + Ingarone, respectively).
Subgroup9. Patients with unresectable disseminated stomach cancer at the age 36-69 years (N = 38) who had not previously received anti-tumor therapy underwent palliative chemotherapy (epirubicin + oxaliplatin + capecitabine) with the addition of immunotherapy (the stabilized composition vs. Refnot + Ingaron) before the beginning of treatment and in between the cycles of chemotherapy. Reliable improvement of long-term results was noted. After 6 months of observation, more pronounced positive effect of therapy was noticed in the group of patients given the stabilized composition (51% vs. 34%).
Subgroup 10. Patients with unresectable pancreatic cancer at the age of 46-79 years (N = 36) who had not previously received anti-tumor therapy had a marginally resectable and unresectable forms. Induction chemotherapy FOLFIRINOX was performed with the addition of immunotherapy (the stabilized composition vs. Refnot + Inharon) before the beginning of treatment and between chemotherapy cycles (5-6 courses every 2 weeks), after which the clinical efficacy of the therapy and tumor resectability were evaluated. As a result, after induction therapy, 8 patients in each group (44%) were operated. After 6 months of observation, half of the patients given the stabilized composition were in the state of clinical remission (22% vs. 6%).

Biochemical analysis of venous blood of the patients (N = 100) performed during two days of drug application demonstrated different dynamics of changes in the level of tumor necrosis factor (TNF) in two groups (see Fig. 1).

In patients given the stabilized composition, a gradual increase in the level of TNF was observed from the moment of the first administration of a single dose with a mild reaching of the peak concentration of TNF (Cmax = 61.56 pg / ml, Tmax = 14 h) after repeated intake of the stabilized composition and retention of a sufficiently high level of TNF for more than 15 hours. On the contrary, subcutaneous injection of Refnot(200.000 IU)resulted in a rapid increase in the concentration of TNF in the blood to maximum (Cmax = 84.76 pg/ml) during several hours, followed by a drop in the level of TNF to 27.66 pg / ml during 5 hours and a slight increase to an average of 31.88 pg/ml in 2 hours after subcutaneous injection of Ingarone(500.000 IU).

During the controlled randomized clinical trials of the dynamics of TNF levels in venous blood with use of the stabilized composition in comparison with the combined pharmacotherapy scheme with the application of Refnot and Ingaron (Refnot 200.000 IU subcutaneously once a day every day and Ingaron 500.000 IU subcutaneously 1 time per day every other day) in patients with disseminated skin melanoma (N = 210), the association of development of adverse reactions with a high level of TNF was established when the combined schemes of pharmacotherapy were used. More often, adverse reactions were recorded on the day of Refnot administration. At the same time, the stabilized composition showed excellent tolerability (incidence of adverse reactions was 7% vs. 31%) with better long-term results.

Technical results of the claimed invention are as follows:
- achievement of the stabilizing effect of cytokines due to the optimally selected composition of the components and their quantity, which allows for storage of interferon gamma and TNF-T under normal conditions without loss of their activity;
- preparation of the stabilized composition based on human recombinant interferon gamma and TNF-T possessing high antitumor, antiviral, antioxidant, immunomodulating, actoprotective and antimutagenic activity;
- extension of the storage temperature range of interferon gamma and TNF-T without loss of their activity;
- minimal adverse effect of the stabilized composition components , including absence of subfebrile states and dyspeptic disorders, which makes it possible to use the composition in complex therapy of oncological diseases, including anti-relapse treatment, for the prevention of cancer and for improving the quality of life of humans and animals.

The following examples illustrate the invention and disclose various aspects of possible implementation of the present invention. The following examples should not be construed as limiting the invention as claimed.

### EXAMPLE 1.Preparation of the stabilized composition with the activity of interferon gamma 50000 IU/g and that of TNF-T 20000U/g.

Use lyophilized interferon gamma and lyophilized TNF-T with the activity of 10.000.000 IU/g and 10.000.000 U/g, respectively. When using lyophilized substances with different activity, it is necessary to recalculate the amount added per 100 g of powder.

Place 20 grams of grape seed powder, 0.6 g of aerosil, 14.5 g of starch, 1.2 g of calcium stearate, 6.0 g of lactose, 6.0 g of microcrystalline carboxymethylcellulose into a mixer. Add 0.5 g of lyophilized interferon gamma and 0.2 g of lyophilized thymosin-alpha 1 tumor necrosis factor. Bring the weight of the resulting mixture to 100 g with mannitol and mix. Place the resulting powder (50000 IU/g for gamma interferon and 20000 U/g for TNF-T) into acid-resistant capsules No. 5. The activity of interferon and TNF-T should be 2500 IU and 1000 U per one capsule, respectively.

Use the obtained capsules No. 5 for the study of antimutagenic properties of the composition. Use a well-known method for chromosome aberration estimation to control the effect of the claimed stabilized composition (see above).

Thus, it has been experimentally confirmed that use of the designed stabilized composition reduces the level of chromosomal aberrations a rising from the effect of cyclophosphamide, a chemotherapeutic drug. Thus, the claimed stabilized composition demonstrates antimutagenic and antioxidant properties.

### EXAMPLE 2.Preparation of the stabilized composition with the activity of interferon gamma 150000 IU/g and that of TNF-T 60000AU/g.

Use lyophilized interferon gamma and TNF-T with the activity of 10.000.000 IU/g and 10.000.000 U/g, respectively. When using lyophilized substances with different activity, it is necessary to recalculate the amount added per 100 g of powder.

Place 25 g of grape seed powder, 1.2 g of aerosil, 22 g of starch, 2.5 g of calcium stearate, 10.05 g of microcrystalline carboxymethylcellulose and 10 g of lactose into a mixer. Add 1.5 g of lyophilized interferon gamma, 0.6 g of lyophilized thymosin alpha 1 tumor necrosis factor. Bring the weight of the resulting mixture to 100 g with mannitol and mix. Place the resulting powder (150000 IU/g for gamma interferon and 60000 U/g for TNF-T) into acid-resistant capsules No. 5. The activity of interferon and TNF-T should be 7500 IU and3000 U per one capsule, respectively.

Use the resulting capsules to confirm the oncoprotective properties of the claimed stabilized composition (see above).

CBA/Lac-Y male mice at the age of 4-5 months weighing 18-22 grams were used. All animals were subdivided into two groups, one of which was used as a control. Animals of the control group were given a placebo placed in capsules No. 5. Animals of the experimental group were given the stabilized compositionplaced in capsules No. 5daily. An oral probe was used to administer the capsules. Capsules with the claimed stabilized composition were given according to the following scheme: administration during one month with two months interval. The total duration of the experiment was 12 months, during which, at equal intervals, blood indices recorded in the experiment were assessed in at least five animals from both groups.

As a result, it was found that use of the stabilized composition in the preventive regimen, including early ontogenesis contributes to a long-term increase in the number of CD11a + and CD11b + lymphocytes, expressing the molecules of LFA-1 and Mac-1, respectively, ascompared to control animals. Thus, it has been established that use of the claimed formulation for preventive purposes does indeed reduce the level of hepatocarcinoma formation, i.e. the claimed composition has an oncoprotective effect.

### EXAMPLE 3.Preparation of the stabilized composition with the activity of interferon gamma and TNF-T250.000 IU/g and 100.000 U/g, respectively.

Place 30 g of grape seed powder, 1.8 g of aerosil, 29.5 g of starch, 3.7 g of calcium stearate, 14.1 g of crystalline carboxymethylcellulose, 14 g of lactose into a mixer. Add 2.5 grams of lyophilized interferon gamma with an activity of 10.000.000 IU/g and 1 g of lyophilized tumor necrosis factor thymosin alpha 1 with an activity of 10.000.00 U/g. Adjust the weight of the resulting mixture to 100 g with mannitol. Stir the resulting 100 g of the mixture. Place the resulting powder into 500 acid-resistant capsules No. 1, 0.2 g each, with an activity of interferon gamma of 50.000 IU per capsule (250000 IU/g) and that of TNF-T20.000 U per one capsule (100.000 U/g).

The resulting capsuleswere given to patients with metastatic colorectal cancer (adenocarcinoma) at the age of 41-71 years (N = 58) who had not received anti-tumor therapy before. Then the chemotherapy was carried out according to the FOLFOX6 scheme with an interval of 2 weeks, during which the immunotherapy was continued. Combined treatment showed efficacy in more than half of patients (55%) after 2 months. After 6 months, the effectiveness of the therapy in the test group given the capsules with the stabilized composition reached 68%, which indicates the effectiveness of the claimed stabilized composition with respect to oncological states of viral etiology. Thus, the claimed stabilized composition has a pronounced antiviral and antitumor effect.

## Claims

1. A stabilized composition possessing antiviral, antitumor, immunomodulating, actoprotective, antimutagenic and antioxidant activity. The composition represents an acid-resistant capsule containing calcium stearate, aerosil, microcrystalline carboxymethylcellulose, starch, lactose, grape seed powder, mannitol and low doses of lyophilyzed human recombinant gamma and tumor necrosis factor-thymosin alpha 1 comprising per 1 g of the composition(wt%):
| | |
|---|---|
| lyophilized human recombinant interferon gamma with activity 10 x 10⁶ IU / g | 0.5 - 2.5 |
| lyophilized recombinant tumor necrosis factor-thymosin alpha 1 with activity 10 x 10⁶ U / g 0.2-1.0 1 | 0.5 - 2.5 |
| grape seed powder | 20-30 |
| calcium stearate | 1.23-3.7 |
| aerosil | 0.6-1.8 |
| microcrystalline carboxymethylcellulose | 6-14.1 |
| starch | 14.5-29.5 |
| lactose | 6-14 |
| mannitol | remaining |

2. The stabilized composition of claim 1, where in the lyophilizedhuman recombinant interferon-gamma is constructed from interferon gamma substance isolated from E. coli strain transformed with plasmid pGIF315

3. The stabilized composition of claim 1, where in the lyophilized recombinant tumor necrosis factor-thymosin alpha is constructed from the substance of tumor necrosis factor-thymosin alpha 1 isolated from Escherichia coli strain-producer transformed with plasmid pThy316.
